(19) Europäisches Patentamt
European Patent Office
Office européen des brevets

(11) Veröffentlichungsnummer : **0 410 191 B1**

(12) # EUROPÄISCHE PATENTSCHRIFT

(45) Veröffentlichungstag der Patentschrift : **20.10.93 Patentblatt 93/42**

(21) Anmeldenummer : **90113023.7**

(22) Anmeldetag : **07.07.90**

(51) Int. Cl.$^5$ : **C07C 205/51,** C07C 205/44, C07C 205/45, C07C 205/53, C07C 201/12, C07C 225/20, C07C 255/10, C07C 211/37, A01N 35/02, A01N 35/06, A01N 33/04

(54) **Neue fluorierte Cyclohexanderivate, Verfahren und Zwischenprodukte zu deren Herstellung und die Verwendung der neuen fluorierten Cyclohexanderivate als Fungizide und Zwischenprodukte.**

(30) Priorität : **22.07.89 DE 3924304**

(43) Veröffentlichungstag der Anmeldung : **30.01.91 Patentblatt 91/05**

(45) Bekanntmachung des Hinweises auf die Patenterteilung : **20.10.93 Patentblatt 93/42**

(84) Benannte Vertragsstaaten : **CH DE FR GB IT LI**

(56) Entgegenhaltungen :
**EP-A- 0 244 613**
**EP-A- 0 317 888**
**DE-A- 2 630 562**
**DE-A- 3 808 276**

(73) Patentinhaber : **BAYER AG**
**D-51368 Leverkusen (DE)**

(72) Erfinder : **Negele, Michael, Dr.**
**Wolfskaul 6**
**D-5000 Köln 80 (DE)**
Erfinder : **Baasner, Bernd, Dr.**
**Wagner-Strasse 83**
**D-5060 Bergisch Gladbach 2 (DE)**

**EP 0 410 191 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue fluorierte Cyclohexanderivate, die mindestens eine Nitro- oder Amino-Gruppe enthalten und vielfältig substituiert sein können, Verfahren und Zwischenprodukte zu deren Herstellung und die Verwendung der neuen fluorierten Cyclohexanderivate als Fungizide, Wirkstoffe gegen Parasiten von Nutztieren und Zwischenprodukte zur Herstellung von biologisch aktiven Substanzen.

Es sind Verfahren bekannt, bei denen man Fluornitroalkane des Typs a)

a) 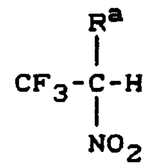

mit einer α,β-ungesättigten Verbindung des Typs b)

b) 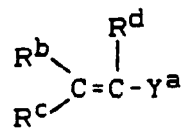

mit $Y^a$ = $COR^e$, $COOR^f$, CN, $NO_2$ oder $SO_3R^g$,
umsetzt und dabei fluorierte Nitroalkylverbindungen des Typs c)

c) 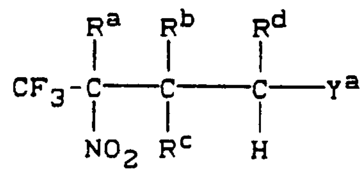

erhält. Die Umsetzung kann in Gegenwart von Alkalimetallfluoriden und einem aprotischen Lösungsmittel (siehe DE-OS 37 39 784) oder in Gegenwart von Aluminiumoxid (siehe DE-OS 38 08 276) erfolgen. Falls Fluornitroalkane des Typs a) mit $R^a$ = Wasserstoff zum Einsatz gelangen, können in unerwünschter Weise auch 2 Mole der Verbindung des Typs b) mit 1 Mol der Verbindung des Typs a) reagieren. Welche Produkte sich dabei bilden ist nicht angegeben.

Es wurden nun neue fluorierte Cyclohexanderivate der Formel (I) gefunden,

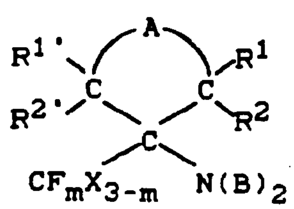 (I),

in der
A für einen der folgenden $C_3$-Alkylenreste

i) 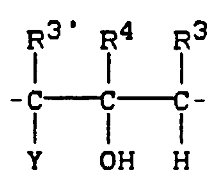 ii) 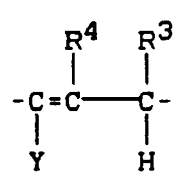

iii) 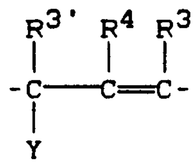 iv) 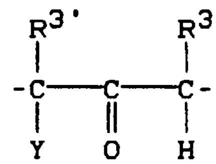

v) 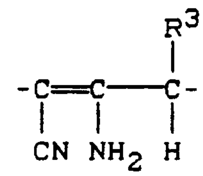 vi) 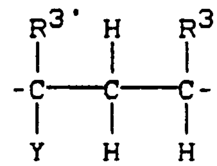

vii) 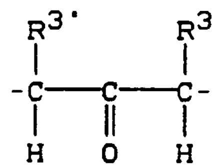 viii) 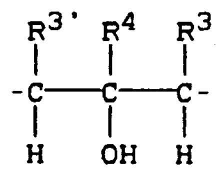

ix) 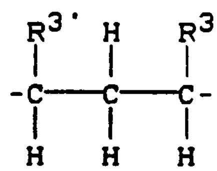 ,

| | |
|---|---|
| B | für Sauerstoff oder Wasserstoff, |
| X | für Wasserstoff, Chlor, Brom oder Methyl, |
| m | für 1, 2 oder 3, |
| $R^1$, $R^2$, $R^3$, $R^{1'}$, $R^{2'}$ und $R^{3'}$ | unabhängig voneinander jeweils für Wasserstoff, Halogen, Cyano, gegebenenfalls substituiertes $C_1$- bis $C_6$-Alkyl oder gegebenenfalls substituiertes $C_6$- bis $C_{10}$-Aryl, |
| $R^4$ | für Wasserstoff, $C_1$- bis $C_6$-Alkyl oder $C_6$- bis $C_{10}$-Aryl |

und

Y für eine $COR^{4'}$-Gruppe mit $R^{4'}$ = Wasserstoff, $C_1$-bis $C_6$-Alkyl oder $C_6$- bis $C_{10}$-Aryl (unabhängig von $R^4$) oder
eine $CO\text{-}OR^5$-Gruppe mit $R^5$ = $C_1$- bis $C_6$-Alkyl oder $C_6$- bis $C_{10}$-Aryl oder
eine $SO_2\text{-}OR^6$-Gruppe mit $R^6$ = $C_1$- bis $C_6$-Alkyl oder $C_6$- bis $C_{10}$-Aryl oder
eine Cyanogruppe oder
eine Nitrogruppe

stehen.

Soweit in Formel (I) als Reste substituierte Alkyl- und/oder Arylreste vorhanden sein können, kommen dabei als Substituenten beispielsweise Nitro-, Halogen-, $C_1$- bis $C_4$-Alkoxy- und Cyano-Gruppen in Frage. Vorzugsweise steht dabei Halogen für Fluor, Chlor oder Brom, insbesondere für Fluor. Als Substituenten an Alkylresten kommen auch aromatische Reste in Frage.

Vorzugsweise sind jeweils $R^1$ und $R^{1'}$, unabhängig davon $R^2$ und $R^{2'}$, und unabhängig davon $R^3$ und $R^{3'}$ identisch.

Bevorzugt sind fluorierte Cyclohexanderivate der Formel (I), bei denen

| | |
|---|---|
| A | für i), ii), iv), vii) oder viii), |
| X | für Wasserstoff oder Chlor, |
| m | für 2 oder 3, |
| $R^1$, $R^2$, $R^3$, $R^{1'}$, $R^{2'}$ und $R^{3'}$ | unabhängig voneinander jeweils für Wasserstoff, Halogen, unsubstituiertes $C_1$- bis $C_6$-Alkyl oder substituiertes $C_6$- bis $C_{10}$-Aryl und |
| Y | für eine $COR^{4'}$-Gruppe mit$^{4'}$ = Wasserstoff, $C_1$-bis $C_6$-Alkyl oder $C_6$- bis $C_{10}$-Aryl (unabhängig von $R^4$) oder<br>eine $CO\text{-}OR^5$-Gruppe mit $R^5$ = $C_1$- bis $C_6$-Alkyl oder $C_6$- bis $C_{10}$-Aryl oder |

eine Cyanogruppe

stehen.

Besonders bevorzugte Einzelverbindungen der Formel (I) sind solche, bei denen die Substituenten folgende Bedeutung haben:

a) A = i), B = Sauerstoff, m = 3, $R^1 = R^2 = R^3 = R^{1'} = R^{2'} = R^{3'}$ = Wasserstoff, $R^4$ = Methyl und Y = $COCH_3$,

b) wie a), jedoch B = Wasserstoff,

c) wie a), jedoch Y = Cyano,

d) A = ii), B = Sauerstoff, m = 3, $R^1 = R^2 = R^3 = R^{1'} = R^{2'} = R^{3'}$ = Wasserstoff, $R^4$ = Wasserstoff und Y = CHO,

e) wie d), jedoch $R^4$ = Methyl,

f) A = iv), B = Sauerstoff, m = 3, $R^1 = R^2 = R^3 = R^{1'} = R^{2'} = R^{3'}$ = Wasserstoff und Y = $COOCH_3$ und

g) A = ix), B = Wasserstoff, m = 3 und $R^1 = R^2 = R^3 = R^{1'} = R^{2'} = R^{3'}$ = Wasserstoff.

Die neuen fluorierten Cyclohexanderivate der Formel (I) können jeweils als ein einheitliches Strukturisomeres, aber auch als ein beliebiges Gemisch verchiedener Strukturisomerer vorliegen.

Die neuen fluorierten Cyclohexanderivate der Formel (I) sind biologisch aktive Verbindungen und/oder Zwischenprodukte zur Herstellung biologisch aktiver Verbindungen. Biologisch aktiv bedeutet dabei beispielsweise, daß die Verbindungen eine gute fungizide Wirksamkeit im Pflanzenschutz bei guter Pflanzenverträglichkeit aufweisen. Ihre besondere Wirksamkeit richtet sich gegen den Erreger des Apfelschorfs.

Die gute Pflanzenverträglichkeit der erfindungsgemäßen fluorierten Cyclohexanderivate in den zur Bekämpfung von Pflanzenkrankheiten notwendigen Konzentrationen erlaubt eine Behandlung von oberirdischen Pflanzenteilen, von Pflanz- und Saatgut und die Behandlung des Bodens. Die Wirkstoffe können in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie in ULV-Formulierungen.

Diese Formulierungen können in an sich bekannter Weise hergestellt werden, beispielsweise durch Vermischen der Wirkstoffe mit Streckmitteln, z.B. flüssigen Lösungsmitteln, unter Druck stehenden verflüssigten Gasen und/oder festen Trägerstoffen, gegebenenfalls unter Verwendung von Hilfsmitteln wie oberflächenaktive Mittel, Emulgiermittel, Dispergiermittel und/oder schaumerzeugende Mittel. Im Falle der Benutzung von Wasser als Streckmittel können beispielsweise auch organische Lösungsmittel als Hilfslösungsmittel angewendet werden. Als flüssige Lösungsmittel kommen beispielsweise in Frage Aromaten, chlorierte Aromaten, chlorierte aliphatische Kohlenwasserstoffe, aliphatische Kohlenwasserstoffe, Alkohole, Ether, Ester, Ketone, Dimethylformamid, Dimethylsulfoxid und/oder Wasser. Als verflüssigte gasförmige Streckmittel oder Trägerstoffe werden solche Flüssigkeiten verstanden, die bei normalem Druck und normaler Temperatur gasförmig sind, beispielsweise Aerosoltreibgase wie niedrige Halogenkohlenwasserstoffe, Butan, Propan, Stickstoff und Kohlendioxid. Als feste Trägerstoffe kommen beispielsweise in Frage natürliche Gesteinsmehle, synthetische Gesteinsmehle, hochdisperse Kieselsäure, Aluminiumoxid und Silikate. Als feste Trägerstoffe für Granulate sind weiterhin von Interesse gebrochene und fraktionierte natürliche Gesteine sowie synthetische Granulate aus anorganischen und organischen Mehlen sowie Granulate aus organischem Material wie Sägemehl, Kokosnußschalen, Maiskolben oder Tabakstengel. Als Emulgier- und/oder schaumerzeugende Mittel kommen beispielsweise nichtionogene und anionische Emulgatoren in Frage, als Dispergiermittel Lignin-Sulfit-Ablaugen und Methylcellulose.

In die Formulierungen können auch Haftmittel wie Carboxymethylcellulose eingesetzt werden.

Die Formulierungen können beispielsweise 0,1 bis 95 Gew.-% Wirkstoff, vorzugsweise 0,5 bis 90 Gew.-%, enthalten. Die erfindungsgemäßen Wirkstoffe können in den Formulierungen in Mischung mit anderen bekannten Wirkstoffen vorliegen, z.B. in Kombination mit anderen Fungiziden, Insektiziden, Akariziden und Herbiziden, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertigen Lösungen, emulgierbaren Konzentraten, Emulsionen, Schäumen, Suspensionen, Spritzpulver, Pasten, löslichen Pulvern, Stäubemitteln und/oder Granulaten, angewendet werden. Die Anwendung kann beispielsweise durch Gießen, Verspritzen, Versprühen, Verstreuen, Verstäuben, Verschäumen oder Bestreichen erfolgen. Es ist ferner möglich, die Wirkstoffe nach dem Ultra-Low-Volume (ULV)-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem großen Bereich variiert werden. Sie liegen im allgemeinen bei 1 bis 0,0001 Gew.-%, bevorzugt bei 0,5 bis 0,001 Gew.%.

Bei der Saatgutbehandlung werden im allgemeinen Wirkstoffmengen von 0,001 bis 50 g/kg Saatgut, bevorzugt 0,01 bis 10 g/kg Saatgut angewendet.

Bei der Behandlung des Bodens sind Wirkstoffkonzentrationen im Bereich von 0,0001 bis 0,1 Gew.-%, bevorzugt 0,0001 bis 0,02 Gew.-% am Wirkungsort anwendbar.

Soweit neue fluorierte Cyclohexanderivate der Formel (I) als Zwischenprodukte zur Herstellung biologisch aktiver Verbindungen eingesetzt werden, können daraus beispielsweise durch Umsetzung mit Chlorameisensäureestern Carbamate oder mit Isocyanaten Harnstoffe hergestellt werden, die insektizide und/oder herbizide Aktivität besitzen.

Die vorliegende Erfindung betrifft weiterhin ein Verfahren zur Herstellung von fluorierten Cyclohexanderivaten der Formel (I), das dadurch gekennzeichnet ist, daß man eine Verbindung der Formel (II)

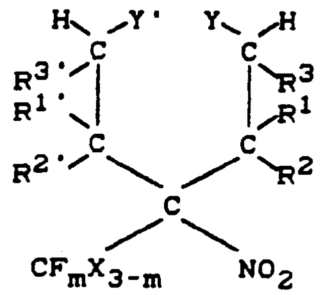 (II),

in der

X, m, $R^1$, $R^2$, $R^3$, $R^{1'}$, $R^{2'}$, $R^{3'}$ und Y die bei Formel (I) angegebene Bedeutung haben und
Y' für eine $COR^4$-, $CO\text{-}OR^5$- oder Cyanogruppe ($R^4$ und $R^5$ wie bei Y definiert) steht,

in Gegenwart einer Base cyclisiert und gegebenenfalls anschließend umderivatisiert.

Als Basen kommen die verschiedensten basisch wirkenden Verbindungen in Frage, beispielsweise Alkalimetallfluoride in einem aprotischen Lösungsmittel, Hydroxide, Carbonate, Alkoholate, Hydride und Amide von Alkali- und Erdalkalimetallen, organische Basen wie Tetraalkylammoniumhydroxide und Anionenaustauscher in basischer Form. Reaktionsbedingungen für die Cyclisierungsreaktion sind beispielsweise Temperaturen im Bereich von -78 bis +150°C, Reaktionszeiten im Bereich von 6 bis 48 Stunden und die Gegenwart eines oder mehrerer Lösungsmittel. Bevorzugt ist die Temperatur im Bereich 0 bis 82°C und das Lösungsmittel Acetonitril.

Die Cyclisierungsreaktion verläuft besonders einfach, wenn bei der eingesetzten Verbindung der Formel (II) Y und/oder Y' für $COR^4$-Gruppe steht und man Verbindungen der Formel (I) mit A = i) erhalten will. In diesem Falle ist es im allgemeinen ausreichend, bei der weiter unten beschriebenen Herstellung der Verbindung der Formel (II) das Reaktionsgemisch länger und/oder bei höherer Temperatur zu rühren als üblich, z.B. über Nacht (ca. 18 Std.) bei am Rückfluß siedendem Acetonitril. Die Gegenwart von Alkalifluorid und einem aprotischen Lösungsmittel reicht dann bereits aus, um ohne weiteres und ohne Umderivatisierung die angegebenen Verbindungen der Formel (I) mit A = i) zu erhalten.

Wenn man die Cyclisierung mit anderen Basen als Alkalifluorid in einem aprotischen Lösungsmittel durchführen möchte, ist es vorteilhaft, bei der Herstellung der Verbindung der Formel (II) diese zu isolieren und die Cyclisierungsreaktion getrennt von der Herstellung der Verbindung der Formel (II) durchzuführen.

Wenn man starke Basen einsetzt, z.B. konzentrierte Alkalihydroxid-Lösungen, Alkoholate, Amide oder Hydride und/oder Y bzw. Y' in der eingesetzten Verbindung der Formel (II) einen stark aktivierenden Rest darstellt, wie $COOR^5$, CN oder CHO, so findet im allgemeinen nach der Cyclisierung eine Dehydratisierung statt. Dann werden Verbindungen der Formel (I) mit A = ii) oder iii) erhalten. Verbindungen der Formel (I) mit A = ii) werden vorzugsweise erhalten, wenn man eine Verbindung der Formel (II) einsetzt mit $R^{3'}$ = Wasserstoff und Verbindungen der Formel (I) mit A = iii) werden im allgemeinen erhalten, wenn man eine Verbindung der Formel (II) mit $R^{3'} \neq$ Wasserstoff einsetzt und $R^3$ eine die Dehydratisierung fördernde Gruppe, z.B. eine Aryl- oder Cyanogruppe, ist.

Verbindungen der Formel (I) mit A = iv) und Y = $COOR^5$ können erhalten werden, wenn man eine Verbindung der Formel (II) mit Y = Y' = $COOR^5$ mit einer stöchiometrischen Menge Alkalimetallalkoholat umsetzt und anschließend hydrolysiert. Decarboxyliert man noch zusätzlich, z.B. durch saure Hydrolyse bei erhöhter Temperatur, so können auch Verbindungen der Formel (I) mit A = vii) erhalten werden.

Verbindungen der Formel (I) mit A = v) können erhalten werden, wenn man eine Verbindung der Formel (II) mit Y = Y' = CN und $R^{3'}$ = Wasserstoff mit einer starken Base, z.B. einem Hydrid oder Amid, in hoher Verdünnung im einem inerten Lösungsmittel, z.B. in einem Dialkylether oder cyclischen Ether, cyclisiert. Eine anschließende saure Hydrolyse kann dann Verbindungen der Formel (I) liefern, bei denen A für iv) mit Y = CN und $R^{3'}$ = Wasserstoff steht

Bei sämtlichen, bisher beschriebenen Cyclisierungs- und Umderivatisierungsreaktionen werden Verbindungen der Formel (I) mit B = Sauerstoff erhalten. Man kann daraus die entsprechenden Verbindungen mit B = Wasserstoff durch eine Hydrierung erhalten.

Eine derartige Hydrierung kann man beispielsweise katalytisch mit Wasserstoff oder durch Umsetzung mit Metallhydriden durchführen. Bei der katalytischen Hydrierung mit Wasserstoff kann man in Gegenwart oder in Abwesenheit von Lösungsmitteln arbeiten. Im allgemeinen ist es vorteilhaft, in Gegenwart eines Lösungsmittels zu arbeiten, da dann eine bessere Kontrolle der exotherm verlaufenden Hydrierung möglich ist.

Als Lösungsmittel kommen beispielsweise inerte organische Lösungsmittel in Frage. Geeignet sind beispielsweise Alkohole wie Methanol, Ethanol, Ethylenglykol und Diethylenglykol, Ether wie Dioxan, Tetrahydrofuran, Ethylenglykolmonomethylether, Ethylenglykolmonoethylether, Ethylenglykoldimethylether, Diethylenglykolmonoethylether und Diethylenglykoldimethylether, gesättigte Kohlenwasserstoffe wie Cyclohexan und Ester wie Essigsäureethylester. Bevorzugte Lösungsmittel sind Methanol und Ethanol. Bei der katalytischen Hydrierung kann der Wasserstoffdruck in weiten Grenzen variieren. Bevorzugt sind Drucke im Bereich 1 bis 30 bar, insbesondere 5 bis 20 bar. Die Reaktionstemperatur kann ebenfalls in weiten Grenzen variiert werden, beispielsweise zwischen 0 und 120°C, vorzugsweise zwischen 10 und 80°C. Im allgemeinen ist es vorteilhaft, für die Hydrierung von 1 Mol einer Verbindung der Formel (I) mit B = Sauerstoff zu der entsprechenden Verbindung mit B = Wasserstoff mindestens 3 Mole Wasserstoff einzusetzen. Geeignete Katalysatoren für die katalytische Hydrierung mit Wasserstoff sind beispielsweise solche, die aus Metallen und/oder Verbindungen von Elementen der 8. Nebengruppe des periodischen Systems der Elemente bestehen oder diese enthalten. Dabei sind die Metalle Ruthenium, Rhodium, Palladium, Platin, Kobalt und Nickel und deren Verbindungen bevorzugt. Derartige Metalle und Metallverbindungen können auch auf Trägermaterialien aufgebracht sein. Metallische Katalysatoren können auch als Skelettkatalysatoren vom Raney-Typ eingesetzt werden. Geeignete Katalysatormengen sind beispielsweise solche von 0,1 bis 15 Gew.-%, bezogen auf die eingesetzte Verbindung der Formel (I) mit B = Sauerstoff.

Parallel zu der zuvor beschriebenen Hydrierung einer Verbindung der Formel (I) mit B = Sauerstoff zu der entsprechenden Verbindung mit B = Wasserstoff können weitere Reaktionen stattfinden oder unterdrückt werden. Bei solchen weiteren Reaktionen kann es sich z.B. um eine Überführung einer im Molekülteil A vorhandenen Carbonylgruppe in eine Hydroxylgruppe und/oder um die Hydrierung einer im Molekülteil A vorhandenen Doppelbindung handeln.

Aus Verbindungen der Formel (I) mit Y = $COOR^5$ oder CN lassen sich durch saure Hydrolyse unter milden Bedingungen (z.B. Temperatur unter 60°C) die entsprechenden Carbonsauren herstellen und daraus durch Decarboxylierung entsprechende Verbindungen der Formel (I) mit A = viii) erhalten.

Aus Verbindungen der Formel (I), bei denen im Molekülteil A eine zentrale Carbonylgruppe vorliegt, lassen sich durch Umsetzung mit Hydrazin die entsprechenden Hydrazone herstellen, aus denen durch die Abspaltung von Stickstoff unter dem Einfluß starker Basen und gegebenenfalls erhöhten Temperaturen Verbindungen der Formel (I) mit A = ix) und B = Wasserstoff erhalten werden können, die statt der ursprünglichen Carbonylgruppe eine Methylengruppe enthalten und bei denen die Nitrogruppe zur Aminogruppe reduziert wurde.

Die für die Herstellung der neuen fluorierten Cyclohexanderivate der Formel (I) als Ausgangsverbindungen benötigten Verbindungen der Formel (II) können erhalten werden, indem man 1 Mol eines Fluornitroalkans der Formel (III)

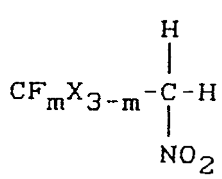

(III),

in der

X und m die bei Formel (I) angegebene Bedeutung haben,

in Gegenwart von Alkalimetallfluoriden und einem polaren, aprotischen Lösungsmittel mit mindestens 2 Molen einer oder mehreren α,β-ungesättigten Verbindungen der Formel (IV) umsetzt

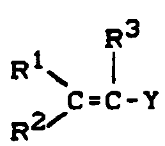

(IV),

in der

$R^1$, $R^2$, $R^3$ und Y die bei Formel (I) angegebene Bedeutung haben.

Im Gegensatz zur DE-OS 3 739 784 wird dabei beispielsweise die Verbindung der Formel (IV) im Überschuß von mindestens 2 Molen vorgelegt und bei tiefer Temperatur, z.B. bei -78 bis +10°C, vorzugsweise bei 0 bis 10°C, mit einem Mol einer Verbindung der Formel (III) umgesetzt. Gegebenenfalls kann man zur Vervollständigung der Reaktion bei erhöhter Temperatur nachrühren, z.B. bei 50 bis 100°C, vorzugsweise bei 70 bis 85°C.

Werden mehrere α,β-ungesättigte Verbindungen der Formel (IV) eingesetzt, insbesondere zwei derartige Verbindungen nacheinander, so lassen sich unsymmetrische Verbindungen der Formel (II) erhalten, d.h. Verbindungen der Formel (II), bei denen $R^1$ und $R^{1'}$, $R^2$ und $R^{2'}$, $R^3$ und $R^{3'}$ und/oder Y und Y' jeweils verschieden voneinander sind.

Eine besondere Verfahrensweise zur Herstellung von Verbindungen der Formel (II) besteht darin, daß man zunächst gemäß der DE-OS eine Verbindung der Formel (III')

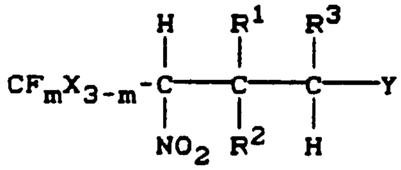

(III')

(Bedeutungen von X, m, $R^1$, $R^2$ und $R^3$ wie bei Formel (I) angegeben)
herstellt, diese reinigt und dann in einem zweiten Schritt mit einer Verbindung der Formel (IV) umsetzt.

Es ist ausgesprochen überraschend, daß mit den neuen fluorierten Cyclohexanderivaten der Formel (I) eine neue Verbindungsklasse mit biologischen Wirkungen zur Verfügung gestellt werden konnte, denn weder die Wirkungen der Stoffe der neuen Verbindungsklasse, noch deren gute Zugänglichkeit konnte aus dem eingangs geschilderten Stand der Technik erwartet werden.

## Beispiele

### Beispiel 1

Herstellung von 4-Trifluormethyl-4-nitropimelinsäuredimethylester (Formel (II), m = 3, $R^1 = R^2 = R^3 = R^{1'} = R^{2'} = R^{3'}$ = Wasserstoff, Y = Y' = $COOCH_3$)

In 150 ml Acetonitril wurden bei 5°C 27 g (0,465 Mol) geglühtes Kaliumfluorid und 86 g (1,0 Mol) Acrylsäuremethylester vorgelegt und langsam unter Rühren innerhalb einer Stunde 60 g (0,465 Mol) Trifluornitroethan zugetropft. Nach dem Ansteigen der Temperatur auf 60°C wurde noch 3 Stunden bei Raumtemperatur nachgerührt. Dann wurde filtriert und der Rückstand mit 100 ml Acetonitril nachgewaschen. Aus den vereinigten Filtraten wurde das Lösungsmittel und nicht umgesetztes Einsatzmaterial im Wasserstrahlvakuum (12 mbar) abgezogen und der Rückstand (98 g) bei 0,05 mbar fraktioniert destilliert. Es wurden 86 g Produkt (= 61 % der Theorie) mit einem Siedepunkt von 124 bis 127°C bei 0,05 mbar erhalten. Das $^1$H-NMR-Spektrum zeigte charakteristische Banden bei δ = 2,38 bis 2,61 ppm (m, 8H, $4CH_2$) und 3,69 ppm (s, 6H, $2OCH_3$) gemessen gegen Tetramethylsilan als inneren Stsndard. Das $^{19}$F-NMR-Spektrum zeigte eine charakteristische Bande bei δ = +7,5 ppm (s, $CF_3$) gemessen gegen $CF_3COOH$ als externen Standard.

### Beispiel 2

Herstellung von 4-Trifluormethyl-4-nitropimelinsäuredinitril (Formel (II), m = 3, $R^1 = R^2 = R^3 = R^{1'} = R^{2'} = R^{3'}$ = Wasserstoff, Y = Y' = CN)

Es wurde verfahren wie in Beispiel 1, jedoch wurden anstelle von 86 g Acrylsäuremethylester 53 g (1,0 Mol) Acrylnitril eingesetzt. Die Aufarbeitung ergab 75 g (= 68 % der Theorie) Produkt mit einem Siedepunkt von 115 bis 118°C bei 0,05 mbar.

Das $^1$H-NMR-Spektrum zeigte charakteristische Banden bei δ = 2,54 bis 2,78 ppm (m, 8H, $4CH_2$) gemessen gegen Tetramethylsilan als inneren Standard. Das $^{19}$F-NMR-Spektrum zeigte eine charakteristische Bande bei δ = +10,4 ppm (s, $CF_3$) gemessen gegen $CF_3COOH$ als externen Standard.

Beispiel 3

Herstellung von 4-Trifluormethyl-4-nitro-7-oxopelargonsäuremethylester (Formel (II) m = 3, $R^1 = R^2 = R^3 = R^{1'} = R^{2'} = R^{3'}$ = Wasserstoff, Y = $COOCH_3$, Y' = $COCH_3$)

In 75 ml Acetonitril wurden bei 5°C 43 g (0,2 Mol) 4-Trifluormethyl-4-nitrovaleriansäuremethylester und 5,8 g (0,1 Mol) geglühtes Kaliumfluorid vorgelegt und langsam unter Rühren mit 17,5 g (0,25 Mol) Methylvinylketon versetzt. Es trat eine Erwärmung bis auf 50°C auf, anschließend wurde 4 Stunden bei Raumtemperatur nachgerührt. Danach wurde filtriert, der Rückstand mit 20 ml Acetonitril nachgewaschen und das Filtrat und die Waschflüssigkeit vereinigt. Daraus wurde das Lösungsmittel und nicht umgesetztes Methylvinylketon im Wasserstrahlvakuum (12 mbar) abgezogen, der Rückstand in 50 ml Methylenchlorid aufgenommen, nacheinander mit 50 ml Wasser, 25 ml 5 gew.-%iger wäßriger Salzsäure und nochmals 50 ml Wasser gewaschen, über Natriumsulfat getrocknet, das Lösungsmittel abgezogen und der Rückstand im Vakuum einer Ölpumpe destilliert. Es wurden 38 g Produkt (= 67 % der Theorie) mit einem Siedepunkt von 102 bis 105°C bei 0,05 mbar erhalten.

Das $^1H$-NMR-Spektrum zeigte charakteristische Banden bei $\delta$ = 2,18 ppm (s, 3H, $CH_3$), 2,41 bis 2,68 ppm (m, 8H, $4CH_2$) und 3,72 ppm (s, 3H, $OCH_3$) gemessen gegen Tetramethylsilan als externen Standard.

Beispiel 4

Herstellung von 4-Trifluormethyl-4-nitro-7-oxocaprylsäurenitril (Formel (II), m = 3, $R^1 = R^2 = R^3 = R^{1'} = R^{2'} = R^{3'}$ = Wasserstoff, Y = CN, Y' = CHO)

In 75 ml Acetonitril wurden bei 5°C 37 g (0,2 Mol) 4-Trifluormethyl-4-nitrovaleronitril und 5,8 g (0,1 Mol) geglühtes Kaliumfluorid vorgelegt und langsam unter Rühren mit 14 g (0,25 Mol) Acrolein versetzt. Die Mischung erwärmte sich auf ca. 40°C und wurde anschließend 3 Stunden bei Raumtemperatur nachgerührt. Danach wurde das Gemisch abfiltriert, der Rückstand mit 20 ml Acetonitril nachgewaschen, das Filtrat und die Waschflüssigkeit vereinigt und daraus das Lösungsmittel und unumgesetztes Acrolein im Wasserstrahlvakuum (12 mbar) abgezogen. Anschließend wurde der Rückstand im 50 ml Methylenchlorid aufgenommen, nacheinander mit 50 ml Wasser, 25 ml 5 gew.-%iger wäßriger Salzsäure und nochmals 50 ml Wasser gewaschen und über Natriumsulfat getrocknet. Nach dem Abziehen des Lösungsmittels wurde im Ölpumpenvakuum destilliert. Es wurden 32 g Produkt (= 72 % der Theorie) mit einem Siedepunkt von 110 bis 112°C bei 0,05 mbar erhalten.

Beispiel 5

Herstellung von 1-Methyl-2-acetyl-4-trifluormethyl-4-nitro-cyclohexan-1-ol (Formel (I), m = 3, B = Sauerstoff, $R^1 = R^2 = R^{1'} = R^{2'}$ = Wasserstoff, A = i) mit $R^3 = R^{3'}$ = Wasserstoff, $R^4 = CH_3$ und Y = $COCH_3$)

In 150 ml Acetonitril wurden bei 5°C 27 g (0,465 Mol) geglühtes Kaliumfluorid und 70 g (1,0 Mol) Methylvinylketon vorgelegt und unter Rühren 60 g (0,465 Mol) Trifluornitroethan innerhalb 1 Stunde zugetropft. Das Reaktionsgemisch erwärmte sich auf 70°C und wurde dann noch bei Raumtemperatur 4 Stunden nachgerührt. Im Reaktionsgemisch lag dann laut gaschromatographischer Untersuchung 5-Trifluormethyl-5-nitrononan-2,8-dion vor (Formel (II), m = 3, $R^1 = R^2 = R^3 = R^{1'} = R^{2'} = R^{3'}$ = Wasserstoff, Y = Y' = $COCH_3$).

Nach weiterem 5-stündigen Rühren bei 80°C war dieses Produkt vollständig zu dem in der Überschrift genannten Produkt cyclisiert. Danach wurde das Reaktionsgemisch abfiltriert, der Rückstand mit 100 ml Acetonitril nachgewaschen, das Filtrat und die Waschflüssigkeit vereinigt und im Wasserstrahlvakuum das Lösungsmittel und nicht umgesetztes Ausgangsmaterial abgezogen. Der Rückstand wurde in 200 ml Methylenchlorid gelöst, mit 100 ml Wasser, danach mit 50 ml 5 gew.-%iger wäßriger Salzsäure und danach nochmals mit 100 ml Wasser gewaschen, über Natriumsulfat getrocknet, das Lösungsmittel abgezogen und so einen öligen Rückstand erhalten, der im Vakuum einer Ölpumpe destilliert wurde. Es wurden 80 g Produkt (= 64 % der Theorie) mit einem Siedepunkt von 112 bis 115°C bei 0,5 mbar erhalten. In der Vorlage erstarrte das Material allmählich und wies einen Schmelzpunkt von 86 bis 87°C auf.

Die aufgenommenen Kernresonanz-Spektren ergaben folgende charakteristische Daten:

$^1H$-NMR: $\delta$ = 1,18 ppm (s, 3H, $CH_3$); 1,24 ppm (dd, 1H, $CH_2$, $^2J$(H-H) = 14,2 Hz, $^3J$(H-H) = 4,7 Hz, $^4J$(H-H) = 3,2 Hz); 1,82 ppm (dt, 1H, $CH_2$, $^2J$(H-H) = 14,2 Hz, $^3J$(H-H) = 4,1 Hz); 2,20 ppm (s, 3H, $CH_3$); 2,43 ppm (dd, 1H, CH, $^3J$(H-H) = 13,5 Hz u. 4,8 Hz); 2,54 - 2,77 ppm (dt u. dq, 4H, $2CH_2$); 3,81 ppm (d, 1H, OH, $^4J$(H-H) = 3,2 Hz) gemessen gegen Tetramethylsilan als internen Standard.

$^{13}C$-NMR (protonenentkoppelt, Zuordnung durch DEPT-Experiment belegt): $\delta$ = 23,4 ppm ($CH_2$); 26,3 ppm ($CH_2$); 28,1 ppm (CH-C=O-); 31,3 ppm ($CH_3$-C-OH); 34,5 ppm ($CH_2$); 52,4 ppm (CH); 67,9 ppm (-C-OH); 91,1 ppm ($CF_3$-C-$NO_2$, J(C-F) = 27 Hz); 122,7 ppm ($CF_3$, J(C-F) = 284 Hz); 211,5 ppm (C=O) gemessen gegen Tetramethylsilan als internen Standard.

$^{19}F$-NMR: $\delta$ = +1,0 ppm (s) gemessen gegen $CF_3COOH$ als externen Standard.

Beispiel 6

Herstellung von 1-Methyl-2-acetyl-4-amino-4-trifluormethylcyclohexan-1-ol (Formel (II) mit m = 3, B = Wasserstoff, $R^1 = R^2 = R^{1'} = R^{2'}$ = Wasserstoff und A = i) mit $R^3 = R^{3'}$ = Wasserstoff, $R^4 = CH_3$ und Y = $COCH_3$)

In 100 ml Methanol wurden 27 g (0,1 Mol) des gemäß Beispiel 5 hergestellten Produktes bei 50 bis 70°C und einem Wasserstoffdruck von 50 bis 60 bar 6 Stunden lang in einem Edelstahlautoklaven an 3 g Palladium auf Kohle (5 Gew.-% Palladium enthaltend) hydriert. Zur Aufarbeitung wurde das Hydriergemisch filtriert und aus dem Filtrat das Lösungsmittel im Wasserstrahlvakuum abgezogen. Der verbleibende Rückstand wurde im Vakuum einer Ölpumpe destilliert und es wurden 15 g (= 63 % der Theorie) des Produktes mit einem Siedepunkt von 68 bis 71°C bei 0,05 mbar erhalten.

Das $^1H$-NMR-Spektrum zeigte charakteristische Banden bei $\delta$ = 1,23 ppm (s, 3H, $CH_3$-C-OH); ca. 1,25 ppm (s, br, 2H, $NH_2$); 1,32 bis 2,08 ppm (mehrere m, 6H, $3CH_2$); 2,28 ppm (s, 3H, $CH_3$-C=O); ca. 3,82 ppm (s, br, 1H, OH) gemessen gegen Tetramethylsilan als inneren Standard.

Das $^{19}F$-NMR-Spektrum zeigte eine charakteristische Bande bei $\delta$ = -6,3 ppm (s, $CF_3$) gemessen gegen $CF_3COOH$ als externen Standard.

Beispiel 7

Herstellung von 1-Formyl-3-trifluormethyl-3-nitrocyclohex-1-en (Formel (II) mit m = 3, B = Sauerstoff, $R^1 = R^2 = R^{1'} = R^{2'}$ = Wasserstoff und A = ii) mit Y = $COR^{4'}$ und $R^3 = R^4 = R^{3'} = R^{4'}$ = Wasserstoff)

In 50 ml Acetonitril wurden bei 50°C 27 g (0,465 Mol) geglühtes Kaliumfluorid und 56 g (1,0 Mol) Acrolein vorgelegt und unter Rühren 60 g (0,465 Mol) Trifluornitroethan in 1 Stunde zugetropft. Das Gemisch erwärmte sich auf 80°C und wurde danach 3 Stunden bei Raumtemperatur nachgerührt. Im Reaktionsgemisch lag dann gemäß gaschromatographischer Untersuchung ein 4:1-Gemisch aus 4-Trifluormethyl-4-nitroheptan-1,7-dial vor (Formel (II) mit m = 3, Y = Y' = CHO, alle anderen Substituenten = Wasserstoff) und 2-Formyl-4-trifluormethyl-4-nitrocyclohexan-1-ol (Formel (I) mit m = 3, B = Sauerstoff, $R^1 = R^2 = R^{1'} = R^{2'}$ = Wasserstoff und A = i) mit Y = CHO, $R^3 = R^4 = R^{3'} = R^{4'}$ = Wasserstoff).

Es wurde weitere 5 Stunden bei 80°C gerührt. Danach war der Ringschluß zum 2-Formyl-4-trifluormethyl-4-nitrocyclohexan-1-ol vollständig.

Danach wurde das Gemisch abfiltriert, der Rückstand mit 100 ml Acetonitril gewaschen, das Filtrat und die Waschflüssigkeit vereinigt und im Wasserstrahlvakuum Lösungsmittel und nicht umgesetztes Ausgangsmaterial abgezogen. Der dabei verbliebene Rückstand wurde in 200 ml Methylenchlorid gelöst, mit 100 ml Wasser, danach 50 ml 5 gew.-%iger wäßriger Salzsäure und danach nochmals 100 ml Wasser gewaschen und über Natriumsulfat getrocknet. Nach dem Abziehen des Lösungsmittels im Wasserstrahlvakuum verblieben 90 g eines Öles, das gemäß gaschromatographischer Untersuchung zu 80 % aus 2-Formyl-4-trifluormethyl-4-nitrocyclohexan-1-ol und zu 20 % des entsprechenden dehydratisierten Produkts (siehe Überschrift) bestand. Während der Destillation dieses Öls erfolgte thermisch die vollständige Dehydratisierung zu dem in der Überschrift angegebenen Produkt. Dieses wurde in einer Ausbeute von 51 g (= 49 % der Theorie) mit einem Siedepunkt von 86 bis 91°C bei 0,05 mbar erhalten.

Aus dem aufgenommenen $^1H$-NMR-Spektrum wurden folgende Shifts und Kopplungskonstanten ermittelt ($^1H$-$^1H$-Kopplungskonstanten J (Hz) in $CDCl_3$ + DMSO-$d_6$, gemessen bei 360.116 MHz)

| Shifts $\delta$ (ppm) | Kopplungskonstanten J (Hz) |
|---|---|
| H an C-2 = 6,883 | $J_{24e}$ = 2,26; $J_{23e'}$ = 2,19; $J_{23a'}$ = 2,85 |
| $H_{3e'}$ = 2,705 | $J_{3e'3a'}$ = (-) 18,33; $J_{3e'4e}$ = 2,26 |
| $H_{3a'}$ = 2,495 | $J_{3a'4e}$ = 6,26 |
| $H_{4e}$ = 2,883 | $J_{4e4a}$ = (-) 14,30 |

| Shifts δ (ppm) | | Kopplungskonstanten J (Hz) |
|---|---|---|
| $H_{4a}$ | = 2,163 | $J_{4a3a'}$ = 10,93; $J_{4a3e'}$ = 6,74 |
| $H_{6e'}$ | = 3,638 | $J_{6e'6a'}$ = (-) 18,25 |
| $H_{6a'}$ | = 2,661 | $J_{6e'2}$ = 2,34 |
| H an C-7 | = 9,488 | |

Die Messungen wurden gegen Tetramethylsilan als inneren Standard durchgeführt.

Das aufgenommene $^{19}F$-NMR-Spektrum wies eine charakteristische Bande bei $\delta$ = +1,8 ppm (s, $CF_3$) auf, gemessen gegen $CF_3COOH$ als externen Standard.

Beispiel 8

Herstellung von 1-Methyl-2-formyl-4-trifluormethyl-4-nitrocyclohex-1-en (Formel (II), m = 3, B = Sauerstoff, $R^1 = R^2 = R^{1'} = R^{2'}$ = Wasserstoff und A = ii) mit Y = $COR^{4'}$, $R^3 = R^{4'} = R^{3'}$ = Wasserstoff und $R^4$ = Methyl)

In 75 ml Acetonitril wurden bei 5°C 40 g (0,2 Mol) 4-Trifluormethyl-4-nitrobutan-2-on und 5,8 g (0,1 Mol) geglühtes Kaliumfluorid vorgelegt und unter Rühren langsam 14 g (0,25 Mol) Acrolein zugesetzt. Das Reaktionsgemisch erwärmte sich auf 40°C, anschließend wurde 3 Stunden bei Raumtemperatur nachgerührt.

Im Reaktionsgemisch lag dann gemäß gaschromatographischer Untersuchung im wesentlichen 4-Trifluormethyl-4-nitro-octan-7-on-1-al (Formel (II) m = 3, Y = CHO, Y' = $COCH_3$ und allen anderen Substituenten = Wasserstoff) vor.

Durch weiteres 5-stündiges Rühren bei 80°C wurde dieses Produkt zu 1-Methyl-2-formyl-4-trifluormethyl-4-nitrocyclohexan-1-ol umgewandelt.

Das Reaktionsgemisch wurde dann abfiltriert, der Rückstand mit 50 ml Acetonitril gewaschen, aus dem mit der Waschflüssigkeit vereinigten Filtrat im Wasserstrahlvakuum das Lösungsmittel und nicht umgesetztes Acrolein abgezogen. Der verbleibende Rückstand wurde in 200 ml Methylenchlorid gelöst, mit 100 ml Wasser, danach 50 ml 5 gew.-%iger Salzsäure und danach nochmals 100 ml Wasser gewaschen und über Natriumsulfat getrocknet. Nach Abziehen des Lösungsmittels wurde der ölige Rückstand im Vakuum einer Ölpumpe destilliert, wobei eine Dehydratisierung zu dem in der Überschrift genannten Produkt stattfand. Es wurden 22 g Produkt (= 43 % der Theorie) mit einem Siedepunkt von 90 bis 94°C bei 0,05 mbar erhalten.

Das $^1H$-NMR-Spektrum zeigte charakteristische Banden bei $\delta$ = 2,16 ppm (s, 3H, $CH_3$); 2,38 bis 2,91 ppm (m, 5H, $CH_2$); 3,66 ppm (dm, 1H, $CH_2$) und 10,5 ppm (s, 1H, CHO) gemessen gegen Tetramethylsilan als internen Standard.

Das $^{19}F$-NMR-Spektrum zeigte eine charakteristische Bande bei $\delta$ = +1,3 ppm (s, $CF_3$) gemessen gegen $CF_3COOH$ als externen Standard.

Beispiel 9

Herstellung von 2-(Methyloxycarbonyl)-4-trifluormethyl-4-nitrocyclohexan-1-on (Formel (I) mit m = 3, B = Sauerstoff, $R^1 = R^2 = R^{1'} = R^{2'}$ = Wasserstoff und A = iv) mit Y = $COOCH_3$ und $R^3 = R^{3'}$ = Wasserstoff)

In 100 ml absolutem Methanol wurden bei Raumtemperatur 30 g (0,1 Mol) des Produktes aus Beispiel 1 vorgelegt und unter Rühren und Eiskühlung 18 g einer 30 gew.-%igen Lösung von Natriummethylat in Methanol (= 0,1 Mol Natriummethylat) zugetropft. Nachdem keine Reaktionswärme mehr frei wurde, wurde das Gemisch über Nacht bei Raumtemperatur gerührt. Anschließend wurde das Reaktionsgemisch mit 6 g Eisessig zur Hydrolyse versetzt und dann in 200 ml Wasser eingerührt. Diese Mischung wurde 3 mal mit je 100 ml Diethylether extrahiert, die vereinigten Etherphasen mit Wasser gewaschen und über Natriumsulfat getrocknet. Danach wurde der Ether im Wasserstrahlvakuum abgezogen und der dabei verbliebene Rückstand im Vakuum einer Ölpumpe destilliert. Es wurden 17 g Produkt (= 63 % der Theorie) mit einem Siedepunkt von 73 bis 79°C bei 0,05 mbar erhalten.

Das $^1H$-NMR-Spektrum zeigte charakteristische Banden bei $\delta$ = 2,39 bis 2,81 ppm (m, 6H, $CH_2$); 3,38 ppm (d, 1H, CH) und 3,89 ppm (s, 3H, $CH_3O$) gemessen gegen Tetramethylsilan als inneren Standard.

Beispiel 10

Herstellung von 4-Trifluormethyl-4-nitrocyclohexan-1-on (Formel (II) mit m = 3, B = Sauerstoff, $R^1 = R^2 = R^{1'}$

= $R^{2'}$ = Wasserstoff und A = vii) mit $R^3$ = $R^{3'}$ = Wasserstoff)

27 g (0,1 Mol) gemäß Beispiel 9 hergestelltes Produkt wurden in 100 ml 20 gew.-%iger Salzsäure 3 Stunden unter Rückfluß gekocht. Nach dem Abkühlen wurde mit Ether extrahiert, der Etherextrakt mit Wasser gewaschen und über Natriumsulfat getrocknet. Nach dem Abziehen des Ethers wurde der Rückstand destilliert. Es wurden 14,8 g (= 70 % der Theorie) des in der Überschrift angegebenen Produktes mit einem Kochpunkt von 50 bis 53°C bei 0,05 mbar erhalten.

Das $^1H$-NMR-Spektrum zeigte charakteristische Banden bei $\delta$ = 2,27 bis 2,75 ppm (m, 8H, $CH_2$) gemessen gegen Tetramethylsilan als inneren Standard.

Beispiel 11

Herstellung von 1-Trifluormethyl-1-aminocyclohexan (Formel (II) mit m = 3, B = Wasserstoff, $R^1$ = $R^2$ = $R^{1'}$ = $R^{2'}$ = Wasserstoff und A = ix) mit $R^3$ = $R^{3'}$ Wasserstoff)

21,1 g (0,01 Mol) gemäß Beispiel 10 hergestelltes Produkt wurden mit 0,3 Mol 85 gew.-%iger Hydrazinlösung und 22 g feingepulvertem Kaliumhydroxid in 100 ml Triethylenglykol 2 Stunden am Rückfluß gekocht. Das überschüssige Hydrazinhydrat wurde abdestilliert bis maximal 190°C. Nach 2 Stunden war die Stickstoffentwicklung abgeschlossen. Das Reaktionsgemisch wurde mit 500 ml Wasser versetzt, mehrfach ausgeethert und die vereinigten Etherphasen über Natriumsulfat getrocknet. Nach dem Abziehen des Ethers wurden 8,8 g (= 53 % der Theorie) des in der Überschrift angegebenen Produktes mit einem Siedepunkt von 112 bis 114°C bei 20 mbar erhalten.

Das $^1H$-NMR-Spektrum zeigte charakteristische Banden bei $\delta$ = 1,3 ppm (breites s, $NH_2$) und 2,16 bis 2,65 ppm (m, 10H, $CH_2$) gemessen gegen Tetramethylsilan als inneren Standard.

Das $^{19}F$-NMR-Spektrum zeigte eine charakteristische Bande bei $\delta$ = -5,8 ppm (s, $CF_3$) gemessen gegen $CF_3COOH$ als externen Standard.

Beispiel 12

Venturia Test (Apfel), protektiv

Eine Wirkstoffzubereitung wurde hergestellt, indem 1 Gew.-Teil des gemäß Beispiel 6 hergestellten Produktes mit 4,7 Gew.-Teilen Aceton und 0,3 Gew.-Teilen Alkylaryl-polyglykolether vermischt und dieses Konzentrat mit Wasser auf eine Konzentration von 10 ppm verdünnt wurde. Zur Prüfung auf protektive Wirksamkeit wurden junge Pflanzen mit der Wirkstoffzubereitung bis zur Tropfnässe bespritzt. Nach dem Antrocknen des Spritzbelages wurden die Pflanzen mit einer wäßrigen Konidiensuspension des Apfelschorferregers (Ventura inaequalis) inokuliert und dann 1 Tag bei 20°C und 100 % relativer Luftfeuchtigkeit in einer Inkubationskabine stehengelassen.

Die Pflanzen wurden dann im Gewächshaus bei 20°C und einer relativen Luftfeuchtigkeit von 70 % aufgestellt.

12 Tage nach der Inokulation erfolgte die Auswertung.

Es ergab sich ein Wirkungsgrad von 62 % gegenüber der unbehandelten Kontrolle.

**Patentansprüche**

1. Neue fluorierte Cyclohexanderivate der Formel (I)

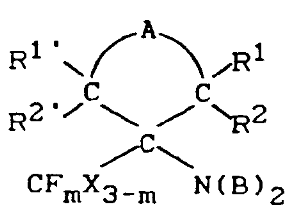

(I),

in der

A für einen der folgenden $C_3$-Alkylenreste

i) 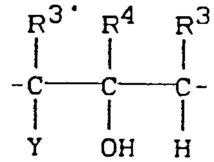 ii) 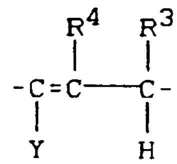

iii) 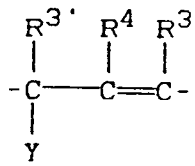 iv) 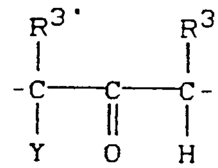

v) 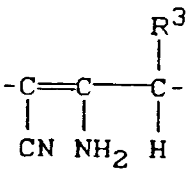 vi) 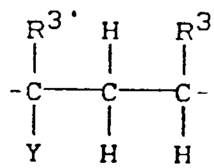

vii) 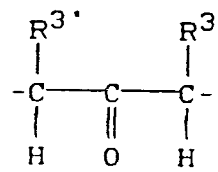 viii) 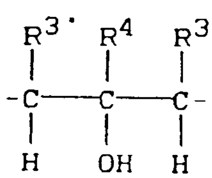

ix) 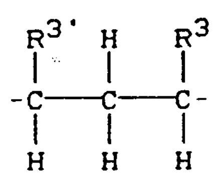 ,

B für Sauerstoff oder Wasserstoff,
X für Wasserstoff, Chlor, Brom oder Methyl,
m für 1, 2 oder 3,
$R^1$, $R^2$, $R^3$, $R^{1'}$, $R^{2'}$ und $R^{3'}$ unabhängig voneinander jeweils für Wasserstoff, Halogen, Cyano, gegebenenfalls substituiertes $C_1$- bis $C_6$-Alkyl oder gegebenenfalls substituiertes $C_6$- bis $C_{10}$-Aryl,
$R^4$ für Wasserstoff, $C_1$- bis $C_6$-Alkyl oder $C_6$- bis $C_{10}$-Aryl
und
Y für eine $COR^{4'}$-Gruppe mit $R^{4'}$ = Wasserstoff, $C_1$-bis $C_6$-Alkyl oder $C_6$- bis $C_{10}$-Aryl (unabhängig von $R^4$) oder
eine $CO$-$OR^5$-Gruppe mit $R^5$ = $C_1$- bis $C_6$-Alkyl oder $C_6$- bis $C_{10}$-Aryl oder
eine $SO_2$-$OR^6$-Gruppe mit $R^6$ = $C_1$- bis $C_6$-Alkyl oder $C_6$- bis $C_{10}$-Aryl oder
eine Cyanogruppe oder
eine Nitrogruppe
stehen.

**2.** Fluorierte Cyclohexanderivate gemäß Anspruch 1, dadurch gekennzeichnet, daß in Formel (I)
A für i), ii), iv), vii) oder viii),
X für Wasserstoff oder Chlor,
m für 2 oder 3,
$R^1$, $R^2$, $R^3$, $R^{1'}$, $R^{2'}$ und $R^{3'}$ unabhängig voneinander jeweils für Wasserstoff, Halogen, unsubstituiertes $C_1$- bis $C_6$-Alkyl oder substituiertes $C_6$- bis $C_{10}$-Aryl und

Y für eine $COR^{4'}$-Gruppe mit $R^{4'}$ = Wasserstoff, $C_1$-bis $C_6$-Alkyl oder $C_6$- bis $C_{10}$-Aryl (unabhängig von $R^4$) oder eine $CO\text{-}OR^5$-Gruppe mit $R^5$ = $C_1$- bis $C_6$-Alkyl oder $C_6$- bis $C_{10}$-Aryl oder eine Cyanogruppe

stehen.

3. Fungizides Mittel, dadurch gekennzeichnet, daß es als Wirkstoff eine Verbindung der Formel (I) und gegebenenfalls übliche Hilfsmittel enthält.

4. Verfahren zur Herstellung von fluorierten Cyclohexanderivaten der Formel (I), dadurch gekennzeichnet, daß man eine Verbindung der Formel (II)

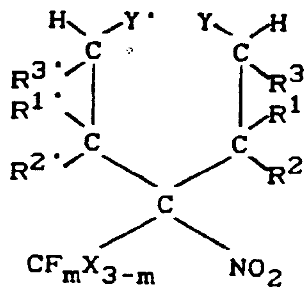

(II),

in der
X, m, $R^1$, $R^2$, $R^3$, $R^{1'}$, $R^{2'}$, $R^{3'}$ und Y die bei Formel (I) angegebene Bedeutung haben und
Y' für eine $COR^4$-, $CO\text{-}OR^5$- oder Cyanogruppe ($R^4$ und $R^5$ wie bei Y definiert) steht,
in Gegenwart einer Base cyclisiert und gegebenenfalls anschließend umderivatisiert.

5. Verfahren nach Anspruch 4, dadurch gekennzeichnet, daß man als Basen Alkalimetallfluoride in einem aprotischen Lösungsmittel, Hydroxide, Carbonate, Alkoholate, Hydride oder Amide von Alkali- oder Erdalkalimetallen, organische Basen oder Anionenaustauscher einsetzt und die Cyclisierungsreaktion bei Temperaturen im Bereich von -78 bis +150°C und in Gegenwart eines oder mehrerer Lösungsmittel durchführt.

6. Verfahren nach Ansprüchen 4 und 5, dadurch gekennzeichnet, daß man das nach der Cyclisierung erhaltene Produkt katalytisch hydriert.

## Claims

1. New fluorinated cyclohexane derivatives of the formula (I)

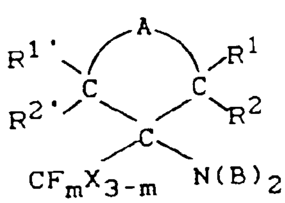

(I),

in which

A represents one of the following $C_3$-alkylene radicals

i) 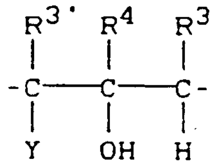 ii) 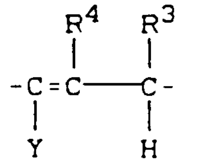

iii) 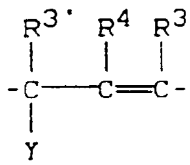 iv) 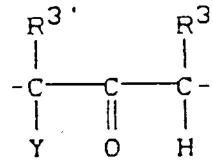

v) 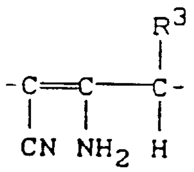 vi) 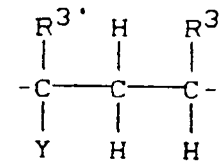

vii) 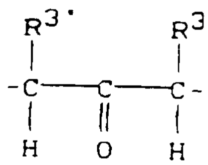 viii) 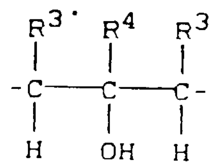

ix) 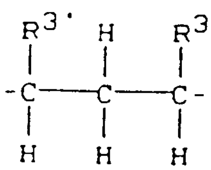 ,

| | |
|---|---|
| B | represents oxygen or hydrogen, |
| X | represents hydrogen, chlorine, bromine or methyl, |
| m | represents 1, 2 or 3, |
| $R^1$, $R^2$, $R^3$, $R^{1'}$, $R^{2'}$ and $R^{3'}$ | independently of one another in each case represent hydrogen, halogen, cyano, optionally substituted $C_1$- to $C_6$-alkyl or optionally substituted $C_6$- to $C_{10}$-aryl, |
| $R^4$ | represents hydrogen, $C_1$- to $C_6$-alkyl or $C_6$- to $C_{10}$-aryl |

and

Y represents a $COR^{4'}$ group in which $R^{4'}$ = hydrogen, $C_1$- to $C_6$-alkyl or $C_6$- to $C_{10}$-aryl (independently from $R^4$) or
a $CO\text{-}OR^5$ group in which $R^5$ = $C_1$- to $C_6$-alkyl or $C_6$- to $C_{10}$-aryl or
a $SO_2\text{-}OR^6$ group in which $R^6$ = $C_1$- to $C_6$-alkyl or $C_6$- to $C_{10}$-aryl or
a cyano group or
a nitro group.

**2.** Fluorinated cyclohexane derivatives according to Claim 1, characterized in that, in formula (I),

| | |
|---|---|
| A | represents i), ii), iv), vii) or viii), |
| X | represents hydrogen or chlorine, |
| m | represents 2 or 3, |
| $R^1$, $R^2$, $R^3$, $R^{1'}$, $R^{2'}$ and $R^{3'}$ | independently of one another in each case represent hydrogen, halogen, unsubstituted $C_1$- to $C_6$-alkyl or substituted $C_6$- to $C_{10}$-aryl, and |
| Y | represents a $COR^{4'}$ group in which $R^{4'}$ = hydrogen, $C_1$- to $C_6$-alkyl or $C_6$- to $C_{10}$-aryl, (independently from $R^4$) or |

a CO-OR$^5$ group in which $R^5$ = $C_1$- to $C_6$-alkyl or $C_6$- to $C_{10}$-aryl or a cyano group.

3. Fungicidal agent, characterized in that it contains a compound of the formula (I) as active compound, and optionally customary auxiliaries.

4. Process for the preparation of fluorinated cyclohexane derivatives of the formula (I), characterized in that, a compound of the formula (II)

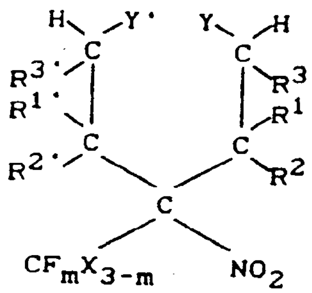

(II),

in which

X, m, $R^1$, $R^2$, $R^3$, $R^{1'}$, $R^{2'}$, $R^{3'}$ and Y have the meaning indicated for formula (I) and

Y' represents a $COR^4$, $CO-OR^5$ or cyano group (where $R^4$ and $R^5$ are defined as for Y),

is cyclized in the presence of a base and where appropriate the product is then converted into another derivative.

5. Process according to Claim 4, characterized in that the bases used are alkali metal fluorides in an aprotic solvent, hydroxides, carbonates, alcoholates, hydrides or amides of alkali metals or alkaline earth metals, organic bases or anion exchange resins and the cyclization reaction is carried out at temperatures in the range from -78 to +150°C and in the presence of one or more solvents.

6. Process according to Claims 4 and 5, characterized in that the product obtained after cyclization is catalytically hydrogenated.

## Revendications

1. Nouveaux dérivés fluorés de cyclohexane de formule (I)

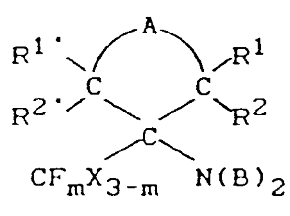

(I)

dans laquelle

A représente l'un des restes alkylène en $C_3$ suivants :

i) 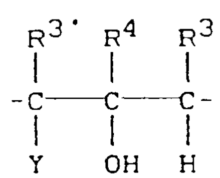 ii) 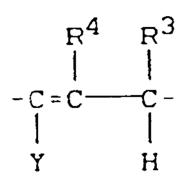

iii) 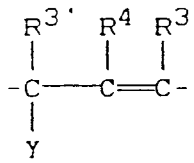 iv) 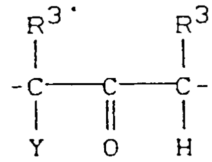

v) 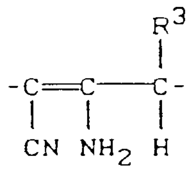 vi) 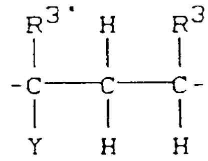

vii) 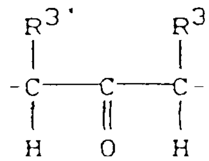 viii) 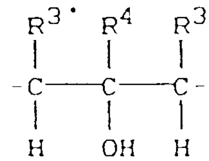

ix) 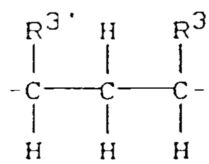 ,

B représente l'oxygène ou l'hydrogène,
X est l'hydrogène, le chlore, le brome ou un groupe méthyle,
m a la valeur 1, 2 ou 3,
$R^1$, $R^2$, $R^3$, $R^{1'}$, $R^{2'}$ et $R^{3'}$ représentent chacun, indépendamment les uns des autres, l'hydrogène, un halogène, un groupe cyano, un groupe alkyle en $C_1$ à $C_6$ éventuellement substitué ou un groupe aryle en $C_6$ à $C_{10}$ éventuellement substitué,
$R^4$ représente l'hydrogène, un groupe alkyle en $C_1$ à $C_6$ ou aryle en $C_6$ à $C_{10}$,
et
Y représente un groupe $COR^{4'}$ dans lequel $R^{4'}$ est l'hydrogène, un radical alkyle en $C_1$ à $C_6$ ou un radical aryle en $C_6$ à $C_{10}$ (indépendamment de $R^4$) ou bien
un groupe $CO$-$OR^5$ dans lequel $R^5$ est un radical alkyle en $C_1$ à $C_6$ ou aryle en $C_6$ à $C_{10}$ ou bien
un groupe $SO_2$-$OR^6$ dans lequel $R^6$ est un radical alkyle en $C_1$ à $C_6$ ou aryle en $C_6$ à $C_{10}$, ou bien
un groupe cyano ou bien
un groupe nitro.

**2.** Dérivés fluorés de cyclohexane suivant la revendication 1, caractérisés en ce que dans la formule (I)
A représente i), ii), iv), vii) ou viii),
X est l'hydrogène ou le chlore,
m a la valeur 2 ou 3,
$R^1$, $R^2$, $R^3$, $R^{1'}$, $R^{2'}$ et $R^{3'}$ représentent chacun, indépendamment les uns des autres, l'hydrogène, un halogène, un groupe alkyle en $C_1$ à $C_6$ non substitué ou un groupe aryle en $C_6$ à $C_{10}$ substitué, et
Y est un groupe $COR^{4'}$ dans lequel $R^{4'}$ est l'hydrogène, un radical alkyle en $C_1$ à $C_6$ ou aryle en $C_6$ à $C_{10}$ (indépendamment de $R^4$) ou bien
un groupe $CO$-$OR^5$ dans lequel $R^5$ est un radical alkyle en $C_1$ à $C_6$ ou aryle en $C_6$ à $C_{10}$ ou bien
un groupe cyano.

**3.** Composition fongicide, caractérisée en ce qu'elle contient comme substance active un composé de formule (I) et, le cas échéant, des substances auxiliaires classiques.

4. Procédé de production de dérivés fluorés de cyclohexane de formule (I), caractérisé en ce qu'on cyclise un composé de formule (II)

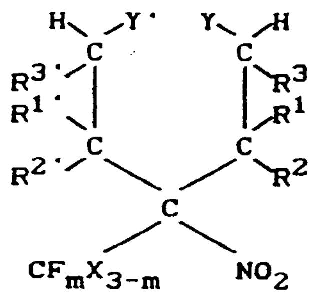

(II)

dans laquelle

X, m, $R^1$, $R^2$, $R^3$, $R^{1'}$, $R^{2'}$, $R^{3'}$ et Y ont la définition indiquée pour la formule (I) et

Y' représente un groupe $COR^4$-, CO-$OR^5$- ou cyano ($R^4$ et $R^5$ étant tels que définis pour Y)

en présence d'une base, et on effectue ensuite le cas échéant une trans-dérivatisation du produit cyclisé.

5. Procédé suivant la revendication 4, caractérisé en ce qu'on utilise comme bases des fluorures de métaux alcalins dans un solvant aprotique, des hydroxydes, des carbonates, des alcoolates, des hydrures ou des amidures de métaux alcalins ou alcalino-terreux, des bases organiques ou des échangeurs anioniques, et on conduit la réaction de cyclisation à des températures comprises dans la plage de -78 à +150°C et en présence d'un ou plusieurs solvants.

6. Procédé suivant les revendications 4 et 5, caractérisé en ce qu'on hydrogène par voie catalytique le produit obtenu après la cyclisation.